# EUROPEAN PATENT APPLICATION

(11) **EP 4 846 926 A1**
(43) Date of publication of application: **02.12.2026**
(21) Application number: 25179716.3
(22) Date of filing: 29.05.2025
(51) Int. Cl.: A61B 5/00, A61B 5/145, A61B 5/1464

(54) **SYSTEM AND METHOD OF UTILIZING RAMAN TO MEASURE FETAL LACTATE**

(71) Applicant: University College Cork, National University Of Ireland, Cork (IE)
(72) Inventor: BURKE, Ray, Cork (IE); MCCARTHY, Fergus, Cork (IE); TOFT, Simon, Cork (IE); YANG, Jason, Cork (IE)
(74) Representative: FRKelly

(57) **Abstract**

The present invention provides a fetal monitoring device a system and method for the identification of biochemical markers associated with hypoxia in a fetus during labour using Raman signals from light reflected from the head of the fetus and identifying Raman signatures corresponding to lactate and other biochemical and physiological changes from the obtained Raman signals.

## Description

### Field of the invention

This invention relates to a system and method for the non-invasive identification of biochemical markers associated with hypoxia in a fetus during labour using optical spectroscopy techniques. The system and method involve obtaining Raman signals from light reflected from the head of the fetus and identifying Raman signatures corresponding to lactate and other biochemical and physiological changes from the obtained Raman signals.

### Background of the invention

Perinatal hypoxia or asphyxia during labour, is the partial or complete lack of oxygen to neonates at the time of labour and delivery. This devastating complication of childbirth is associated with up to 60% of babies dying and represents a significant unmet clinical need in maternal and infant care. Intra-partum hypoxia can lead to several serious medical conditions affecting the neurological system, resulting in cases such as Hypoxic Ischemic Encephalopathy (HIE) and other brain injuries that may lead to paralysis and severe brain damage causing debilitating cognitive delays, developmental problems, and motor impairments.

All babies born by vaginal delivery are potentially at risk of hypoxic injury. Suspected hypoxic injury assessed by current heart rate monitoring instruments, has been a long-standing and common problem managed in labour wards by obstetricians and midwives globally.

Current methods of fetal monitoring during labour include cardiotocography (CTG; fetal heart rate monitoring), the current gold standard and Fetal Blood Sampling (FBS). These technologies are limited by technical challenges such as the necessity for advanced cervical dilation, requirement for regional analgesia or volume of blood required to ascertain fetal pH. Use of computerised interpretation of CTG in women who have continuous electronic fetal monitoring in labour also has been shown to not improve clinical outcomes for mothers or babies (Brocklehurst et al, Lancet 2017). As a result, inadvertent Caesarean sections are performed due to false concerns around CTG readings. Furthermore, misinterpretation, or inability to interpret the CTG, resulting in hypoxic brain injury is a cause of litigation exceeding 1 billion pounds in the UK annually.

There is thus a need for continuous or intermittent, in-utero monitoring of the hypoxic state of the fetus, by means of a device which can preferably be applied locally, and non-invasively to the scalp of the fetus during labour, to detect and through clinical intervention prevent fetal hypoxic brain injury. In addition, accurate detection of fetal hypoxia will ensure that caesareans sections are performed only when absolutely needed. This contrasts with the current situation where reliance on fetal heart rate monitoring which has a poor sensitivity and specificity for fetal hypoxia results in high levels of Caesarean section incorrectly performed for suspected fetal distress.

Such a solution would be applicable to all vaginal births, improving the degree of accuracy and reliability of fetal monitoring when compared to the current gold standard. Given that 75% of labour cases occur gradually, a preventative monitoring approach is ideal at this point in the patient pathway and will result in positive patient outcomes, an improved level of maternity care and a long-term reduction in patient care costs in both mother and child.

It is therefore an object of the present invention to provide an alternative and improved apparatus and method for monitoring for and detecting fetal hypoxia.

### Summary of the invention

According to a first aspect of the present invention there is provided a fetal monitoring device comprising an optical emitter operable to emit excitation light at a wavelength of between 500nm-900nm onto vascularised tissue on the head of a fetus; an optical detector operable to detect Raman signals in the reflected light that are generated through Raman scattering of the excitation light; and a processor operable to convert the Raman signal data from the reflected light into correlated real time data regarding one or more biochemical markers to enable an assessment of fetal wellbeing during labour.

Preferably, the optical emitter and/or the optical detector comprise one or more optical fibres.

Preferably, the emitter and/or the detector optical fibres are arranged in an array.

Preferably, the fetal monitoring device comprises one or more lenses arranged to focus the excitation light onto a capillary bed of the vascularised tissue.

Preferably, the fetal monitoring device comprises one or more optical deflectors arranged to direct the excitation light towards the vascularised tissue.

Preferably, the fetal monitoring device comprises an optical shield arranged to isolate the detector from background electromagnetic radiation.

Preferably, the fetal monitoring device comprises one or more filters arranged to filter the excitation light and/or the reflected light.

Preferably, the fetal monitoring device comprises a camera arranged to image the vascularised tissue on the head of the fetus.

Preferably, the fetal monitoring device comprises a housing at least partially containing the optical emitter and optical detector.

Preferably, the fetal monitoring device comprises one or more sensors operable to measure one or more of temperature, heat flow, SpO2, PCO2, PO2, and heartrate.

Preferably, the processor is operable to convert the Raman signal data from the reflected light into measurements of one or more of blood and tissue lactate level and blood and tissue pH level.

Preferably, the processor utilises an algorithm to convert the Raman signal data into the correlated real time data regarding the one or more biological markers.

Preferably, the algorithm is arranged to effect background correction of the Raman signals.

Preferably, the background correction is effected using one or more of lock-in detection, time-resolved spectroscopy, multi-wavelength excitation and data processing for lactate quantification.

Preferably, the optical detector is operable to additionally detect autoflourescence of NAD(P)H in the vascularised tissue; and the processor is operable to convert the autoflourescence data from the reflected light into correlated real time data regarding one or more of the biochemical markers.

According to a second aspect of the present invention there is provided a method of in-utero monitoring of fetal levels of selected biochemical markers, the method comprising the steps of emitting excitation light at a wavelength of between 500nm-900nm onto vascularised tissue on the head of a fetus; detecting Raman signals in light reflected from the vascularised tissue; and converting the Raman signal data from the reflected light into correlated real time data regarding one or more biological markers.

Preferably, the method comprises the step of converting the Raman signal data from the reflected light into measurements of one or more of blood lactate level, blood pH level, blood oxygen saturation, blood carbon dioxide level, body temperature, hydration and heart rate.

Preferably, the method comprises the step of focussing the excitation light onto a capillary bed of the vascularised tissue.

Preferably, the method comprises the step of emitting the excitation light from a plurality of locations and/or detecting the reflected light from a plurality of locations.

Preferably, the method comprises the step of passing the excitation light and/or the reflected light through one or more filters.

Preferably, the method comprises the step of utilising an algorithm to convert the Raman signal data into the correlated real time data regarding the one or more biological markers.

Preferably, the method comprises the step of effecting background correction of the Raman signals.

Preferably, the background correction is effected by using one or more of lock-in detection, time-resolved spectroscopy, multi-wavelength excitation and data processing for lactate quantification.

Preferably, the method comprises the step of using the optical detector to additionally detect autoflourescence of NAD(P)H in the vascularised tissue; and converting the autoflourescence data from the reflected light into correlated real time data regarding one or more of the biochemical markers.

### Brief description of the drawings

The present invention will now be described with reference to the accompanying drawings, in which:
Figure 1 illustrates a schematic sectioned side elevation of a fetal monitoring device according to a first embodiment of the present invention;
Figure 2 illustrates a modification to the fetal monitoring device shown in Figure 1;
Figure 3 illustrates an alternative modification to the fetal monitoring device shown in Figure 1;
Figure 4 illustrates a schematic sectioned side elevation of a fetal monitoring device according to a second embodiment of the present invention;
Figure 5 illustrates a modification to the fetal monitoring device shown in Figure 4;
Figure 6 illustrates a schematic end view of an alternative arrangement of optical fibres which may be employed in the fetal monitoring device of the invention;
Figure 7 illustrates a modification to the arrangement of optical fibres shown in Figure 6;
Figure 8 illustrates a still further alternative arrangement of optical fibres;
Figure 9 illustrates a modification to the arrangement of optical fibres shown in Figure 8;
Figure 10 illustrates a schematic sectioned side elevation of a fetal monitoring device according to a fifth embodiment of the present invention;
Figure 11 illustrates a modification to the fetal monitoring device shown in Figure 10;
Figure 12 illustrates a perspective view of the fetal monitoring device of the claimed invention including an applicator and a filter forming a part thereof;
Figure 13 illustrates an alternative perspective view of the device as shown in Figure 12;
Figure 14 illustrates a perspective view of the applicator shown in Figures 12 and 13 in isolation;
Figure 15 illustrates an alternative perspective view of the applicator as shown in Figure 14;
Figure 16 illustrates a perspective view of the fetal monitoring device of the claimed invention including an alternative applicator and filter forming a part thereof;
Figure 17 illustrates an alternative perspective view of the device as shown in Figure 16;
Figure 18 illustrates a perspective view of an outer sleeve of the applicator shown in Figures 16 and 17 in isolation;
Figure 19 illustrates an alternative perspective view of the outer sleeve shown in Figure 18;
Figure 20 illustrates a perspective view of an inner body of the applicator shown in Figure 16;
Figure 21 illustrates an alternative perspective view of the inner body;
Figure 22 illustrates an end view of the inner body; and
Figure 23 illustrates a schematic representation of a fetal monitoring device according to a fifth embodiment of the present invention.

### Detailed description of the drawings

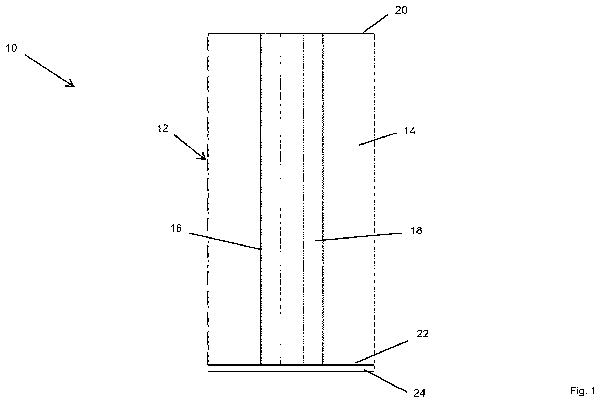

Referring now to Figures 1 to 3 of the accompanying drawings there is illustrated a first embodiment of a fetal monitoring device, generally indicated as 10, for use in real time in utero monitoring of fetal bio-signs, in particular blood lactate and pH in order to monitor the health of the fetus in particular in relation to hypoxia during labour. Using Raman spectroscopy focused on specific spectral wavelengths linked to key biochemical markers the monitoring device 10 can continuously or intermittently measure key parameters, primarily blood lactate level and blood pH level, but optionally also blood oxygen saturation, blood carbon dioxide level, body temperature, hydration and heart rate.

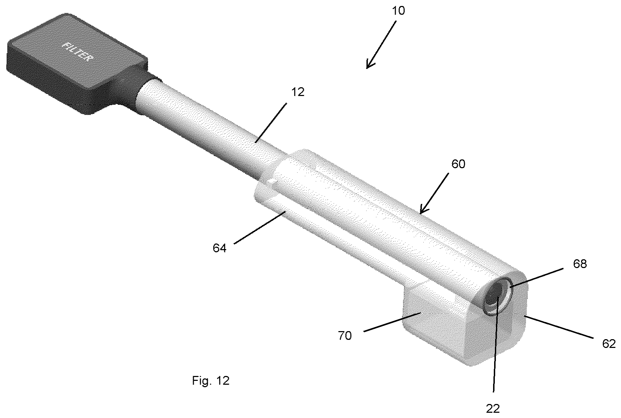

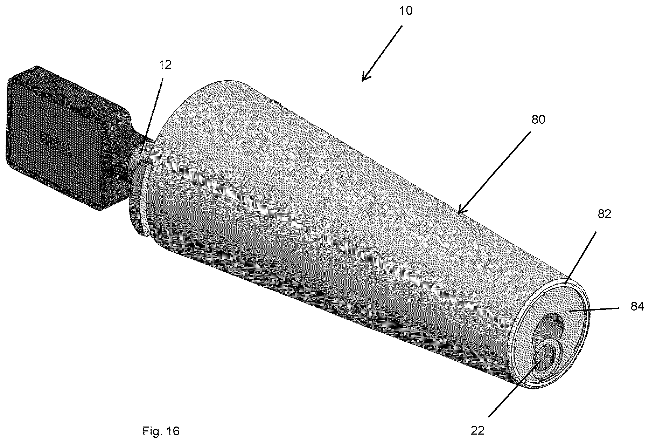

The monitoring device 10 is located on or adjacent the fetal scalp during labour at the time of routine vaginal examination by a health professional to provide a real-time readout of lactate/pH levels to provide visibility and thus reassurance to physicians and midwives on fetal status. Figures 12 and 16 illustrates exemplary applicators for the monitoring device 10, as described in detail hereinafter, which may be employed to position the monitoring device 10 on or adjacent the head of the fetus to effect the in utero monitoring thereof. It will be appreciated that these are exemplary applicators and alternative forms may be employed. The monitoring device 10 allows the non-invasive assessment of the lactate and/or pH levels within the capillary bed at a depth of approximately 2mm under the fetal skin. It is clinically universally accepted that this lactate/pH measurement reflects fetal systemic acid base balance and predicts adverse neonatal outcomes. The monitoring device 10 accesses the capillary bed non-invasively via Raman spectroscopy, essentially focussing a defined wavelength of excitation light onto the scalp and analysing the spectra of the reflected light for signatures of particular biochemical markers indicative of conditions such as hypoxia. The monitoring device is operable to utilise any suitable form of Raman spectroscopy, for example but not limited to inverse spatially offset Raman spectroscopy (iSORs), as described in detail hereinafter.

The device 10 comprises a probe 12 including a housing 14 which is cylindrical in the illustrated embodiment but may be of any other suitable shape and configuration, and which contains an excitation fibre 16 and a detection fibre 18 extending longitudinally through the housing 14 from an upper end 20 to a lower end 22. The excitation fibre 16 and detection fibre 18 comprise optical guides, most preferably each comprising a length of fibre optic cable capable of transmitting optical signals between the upper and lower ends 20, 22. The housing 14 is thus preferably formed from an optically opaque material to prevent light leakage, but preferably also a material which is biocompatible, and may for example comprise a plastic or steel. The distance between the excitation fibre 16 and the detection fibre 18 at the lower end 22 is operationally significant, with the fibres 16, 18 preferably being confocal. However it will be appreciated, as exemplified in further embodiments described hereinafter, that alternative arrangements of the fibres 16, 18 may be employed, in particular by utilising multiple excitation and/or detection fibres in various arrays. The lower end 22 of the probe 12 is preferably covered by a window or lens 24 which may for example be formed from fused silica, sapphire or other suitable optically transparent material. The lens 24 is optional but provides a protective function, and may also be adapted to focus and/or direct light emitted from the probe 12 to a subcutaneous depth in use, for example a depth of 2mm below the surface of the skin.

In use excitation light having a wavelength of between 500nm and 900nm, more preferably between 600nm and 850nm is delivered from a suitable source into the excitation fibre 16 at the upper end of the probe 12. The preferred wavelength is 785nm. The source of the excitation light may for example be a laser (not shown) but could be any other suitable alternative. While the excitation light source is remote from the probe 12, in particular when supplied from a laser, it is envisaged that alterative embodiments of the monitoring device 10 may include a suitable local source on or adjacent the probe 12, for example in the form of a laser diode or super LED. It will be appreciated that a suitable flexible tether (not shown) may couple the probe 12 to the laser or other light source, in order to allow the probe 12 to be located on or adjacent the head of the fetus. The probe 12 may also comprise a camera (not shown) or other imaging device to facilitate remote visualisation of the head of the fetus to facilitate accurate position of the lower end 22 of the probe 12, and for example to reduce or eliminate outer layer interference from hair or the like. A flushing channel (not shown) may be provided in the housing 14 to allow water or the like to be directed onto the head of the fetus to clean away any contaminants that might otherwise negatively affect the operation of the probe 12. The lower end 22 may also be provided with an optical shield (not shown) in order to reduce or eliminate the incursion of background environmental light which again might otherwise negatively affect the operation of the probe 12.

Once the probe 12 is correctly positioned excitation light is transmitted along the excitation fibre 16 from the upper end 20 to the lower end 22 where it is emitted, through the lens 24 if provided, onto an area of vascularised tissue on the head of the fetus (not shown). The optional camera (not shown) may be used to ensure correct targeting of the excitation light. The excitation light will then experience Raman scattering through interacting with, in particular, lactate molecules in the vascularised tissue, imparting a Raman signal or signature to the light that is reflected from the vascularised tissue. This Raman signal is in the form of a shift in the energy of the photons of the excitation light. The reflected light then passes into the detection fibre 18, travelling from the lower end 22 to the upper end 20, back along the tether (not shown) and ultimately to a spectrometer (not shown) where the Raman spectra can be detected and then analysed by a processor (not shown) forming part of the monitoring device 10. As the power of the excitation light is low, for example in the region of mW, and the power of the reflected light used to detect the Raman signals is even lower, for example in the region of µW with very low levels of Raman photons, it is important to filter the reflected light and/or the excitation light in order to enhance the Raman signal, which is orders of magnitude lower than the background luminescence and is thus not an obvious candidate for detecting biochemical markers such as lactate. The monitoring device 10 thus preferably comprises one or more filters (not shown in Figure 1) for example one or more bandpass filters arranged to filter the excitation light to wavelengths suited to Raman scattering by lactate, and/or one or more long pass filters arranged to filter the reflected light, for example to only permit the passage of photons above 800nm. These filters may be located on the probe 12 or remotely.

The spectrometer (not shown) then produces a Raman spectrum from the reflected light which will include peaks at certain wavelengths which are indicative of a change in lactate level in the fetal tissue. This data can utilised by a data processing algorithm running on the processor of the monitoring device 10 as a reference against which real time reflected light is compared as part of a larger data processing procedure in order to present meaningful information to the medical personal regarding the status of the fetus.

In analysing the Raman signals in the reflected light the processor algorithm in its most basic form may employ multiple regression models that are well understood to extract the signal from the background, smooth the signal and normalise the signal to get the maximum sensitivity. These conventional approaches can be customised to the exact application and may also provide additional information about the sampling process or provide basic data that can be combined with other sensor modalities or machine learning models to give better sensitivity and specificity. Machine learning (ML) models may also be employed to identify the biomarker signals and fuse them at different stages in the signal processing to ensure of the signal integrity , stability , sensitivity and specificity are optimised. These machine learning models are trained and tuned using the multiple or multimodal data sets and tested and trained on large test data sets. These ML models will be custom to the specific sensor embodiment and could also be custom to the patient considering the many measurement modes employed.

Specific detection schemes may also be employed by the monitoring device 10 to improve the signal quality compared to standard spectrometer-based methods and offer much higher data collection rates. Such methods can enhance the signal quality for Raman lactate sensing, as well as sensing of other physiologically relevant biomarkers using Raman spectroscopy. Lock-in detection and time-resolved Raman spectroscopy can eliminate the need for a high sensitivity spectrometer. The Raman signal is spectrally filtered and measured with at least one photodetector (not shown). This can offer significantly faster measurement speeds and a higher signal to noise ratio. SERDS eliminates the need for advanced background removal algorithms that can introduce noise to the Raman signal by using a hardware-based background removal method. A brief description of each of these detection schemes is provided below.

### 1) Lock-in detection

The Raman signal is generally orders of magnitude weaker than the background signal. However, the Raman effect is almost instantaneous whereas the background is predominately generated by fluorescence emission on a longer time scale. Because of this difference in the response time, lock-in detection and amplification can effectively isolate and amplify the Raman signal. This can significantly increase the signal to noise ratio. The lock-in signal amplitude is directly correlated with lactate concentration.

The Raman excitation source (e.g. laser) can be amplitude modulated at the reference frequency and the Raman signal extracted in a defined frequency band around the reference frequency. The lock-in detection can use a dual-phase modulation scheme with two orthogonal reference signals. The Raman signal of interest can be spectrally isolated with a bandpass filter at the relevant wavelength of the lactate peak and measured with a discrete photodetector with a fast response or with a dispersive element and an array detector, such as an SiPM or SPAD array.

There are several benefits compared with time-gated Raman spectroscopic methods. For example this method does not require an ultra-fast laser (typically 100ps) and a long duty cycle to avoid/minimise fluorescence from the previous excitation pulse. For tissue spectroscopy, a NIR excitation wavelength (e.g., 785nm or 850nm) is required for the tissue penetration depth. Time-gated Raman spectroscopy using SPAD arrays typically have a very low sensitivity in the NIR whereas discrete photodetectors can have much higher sensitivity.

### 2) Time-resolved Raman spectroscopy

Because of the near-instantaneous response of the Raman effect, the Raman signal can be temporarily isolated from the fluorescence dominated background. Using a boxcar averager and a pulsed (modulated) excitation source, the weak Raman signal can be averaged over a number of duty cycles to increase the signal-to-noise ratio.

The boxcar averager captures the Raman signal in a defined temporal window synchronised to the excitation source. The background, or DC offset, is calculated in a temporal window when the excitation source is off.

The excitation source can be operated at a duty cycle (repetition frequency) that is higher than the fluorescence lifetime, which means that the fluorescence background can be considered constant. This is an advantage over typical time-resolved Raman spectroscopy, which requires a low duty cycle to avoid fluorescence from the previous excitation cycle and a short pulse duration (typically 100ps) to temporally isolate the Raman signal from the fluorescence background.

Another advantage is that the signal can be calculated from a spectral window around the Raman signal of interest. This spectral window can be isolated with a bandpass filter at the relevant wavelength and measured with a discrete photodetector with a fast response.

### 3) Multi-wavelength excitation

The Raman signal from lactate and other relevant chromophores can be separated from the background using two or more excitation sources with slightly shifted emission wavelengths. This technique is called shifted excitation Raman difference spectroscopy (SERDS) and has been demonstrated to remove signals from fluorescence, ambient light and optical components (including the optical fibre), while retaining the Raman signals from the sample.

The theory behind SERDS is that a small shift in excitation wavelength will cause a small shift in the Raman emission bands but will not affect the broad fluorescence dominated background. It is therefore possible to calculate the background free (or reduced) Raman spectrum as the difference between two spectra with shifted excitation wavelengths.

This allows the lactate spectral features to be isolated with accurate hardware-based baseline subtraction.

The typical shift in excitation wavelength is on the order of 0.1-1 nm. This can be achieved with e.g. temperature tuning or laser cavity tuning. For example, two similar laser diodes operated at different temperatures. SERDS can be fibre-coupled as required for this application.

### 4) Data processing for lactate quantification in Raman spectroscopy

A data processing pipeline is demonstrated for lactate quantification from Raman spectroscopy, consisting sequentially of:
1. Spectral Truncation
2. Cosmic Ray removal
3. Detector dark noise removal (per measurement)
4. Savitzky-Golay smoothing filter (per measurement)
5. Background subtraction via "bubblefill" or other filter (per measurement)
6. Mean, standard deviation of spectra across all measurements
7. Average value and standard deviation over region of interest (ROI)

The above assumes that the spectrometer output has been calibrated relative to the excitation source and instrument response.

The monitoring device 10 is preferably also capable of multimodal operation in order to additionally monitor autoflourescence of nicotinamide adenine dinucleotide (NAD(P)H) The monitoring device 10 may also include one or more sensors operable to monitor temperature, heat flow, pCO₂ (partial pressure of carbon dioxide), and pO₂ (partial pressure of oxygen) which data can be used to augment the measurement of the principle components or key measured parameters of lactate and pH to facilitate improved data processing by the processor algorithm to thereby provide supporting data to the clinicians.

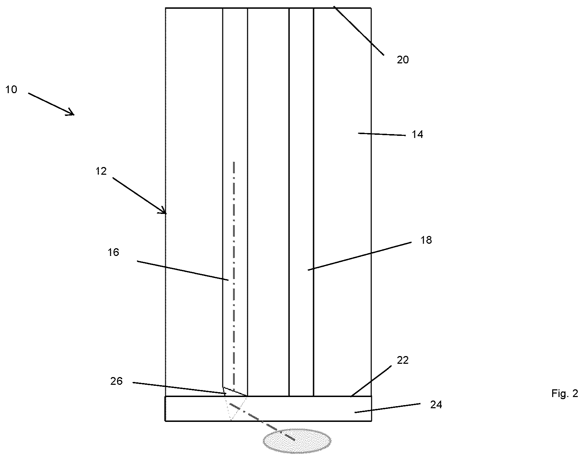

Turning now to Figure 2 there is illustrated a modification to the monitoring device 10, whereby the excitation fibre 16 is provided with an angled prism 26 on the free end of the excitation fibre 16 at the lower end 22. The prism 26 is arranged to deflect the excitation light exiting the excitation fibre 16 to direct it towards a target area on the fetal scalp which is directly beneath the end of the detection fibre 18.

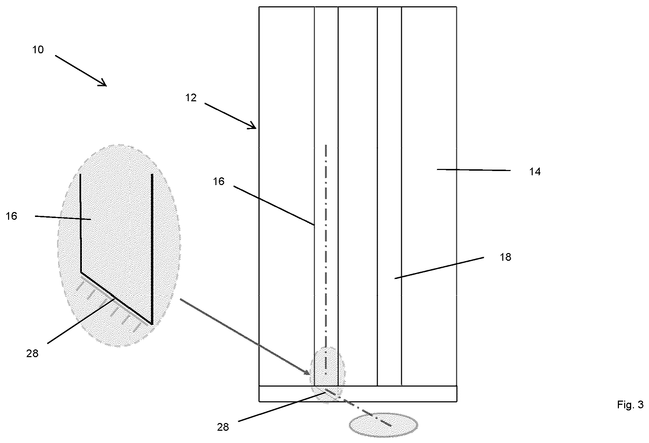

Figure 3 illustrates an alternative modification to the monitoring device 10, whereby the excitation fibre 16 has a cleaved and polished free end 28 at the lower end 22. The cleaved end 28 of the excitation fibre 16 is provided with a reflective coating, for example gold, silver or aluminium, which will again function to direct the exiting light towards a target area on the fetal scalp which is directly beneath the end of the detection fibre 18.

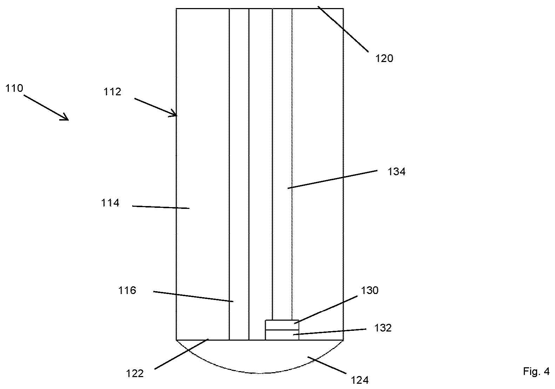

Figure 4 illustrates a second embodiment of a fetal monitoring device according to the present invention, generally indicated as 110. In this second embodiment like components have been accorded like reference numerals and unless otherwise stated perform a like function. The device 110 comprises a probe 112 including a housing 114 which is cylindrical and which contains an excitation fibre 116 extending longitudinally through the housing 114 from an upper end 120 to a lower end 122. The lower end 122 of the probe 112 is preferably covered by a window or lens 124 which may be dome shaped. In place of the detector fibre of the first embodiment the monitoring device 110 comprises a discrete detector 130 which may be in the form of a SiPM, SPAD or CMOS sensor. Upstream of the detector 130 is a filter 132 which may be a long pass or bandpass optical filter. The light reflected from the fetal scalp is therefore passed through the filter 132 to the detector 130 which is operable to achieve local detection of the Raman signals which are then transmitted via electrical connectors 134 to a processor which may be located remotely of the probe 12, for example in an external console or the like on which the real time analysis can be displayed.

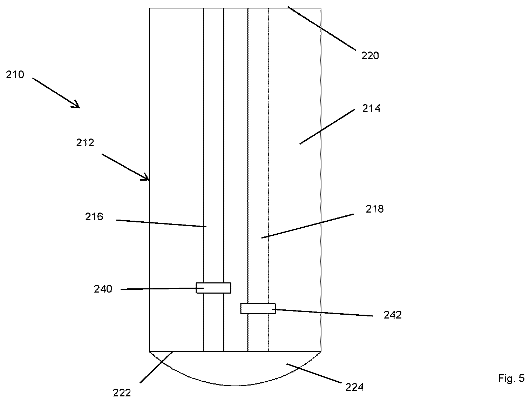

Turning to Figure 5 there is illustrated a third embodiment of a fetal monitoring device according to the present invention, generally indicated as 210. In this third embodiment like components have been accorded like reference numerals and unless otherwise stated perform a like function. The device 210 comprises a probe 212 including a housing 214 which is cylindrical and which contains an excitation fibre 216 and a detection fibre 218 extending longitudinally through the housing 214 from an upper end 220 to a lower end 222. A dome shaped lens 224 covers the lower end 222. An excitation bandbass filter 240 is provided on the excitation fibre 216 while a signal long pass filter 142 is provided on the detection fibre 218. Thus the excitation light is filtered before being directed onto the fetal scalp, while the reflected flight is also filtered before being transmitted to a spectrometer (not shown) for analysis and processing as hereinbefore described.

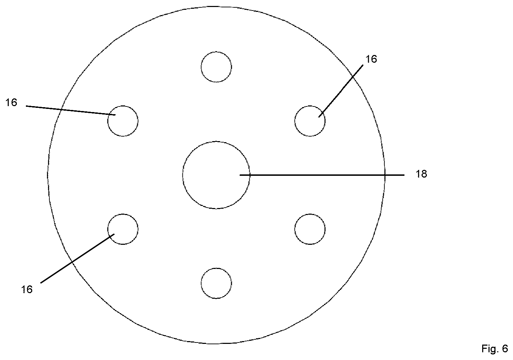

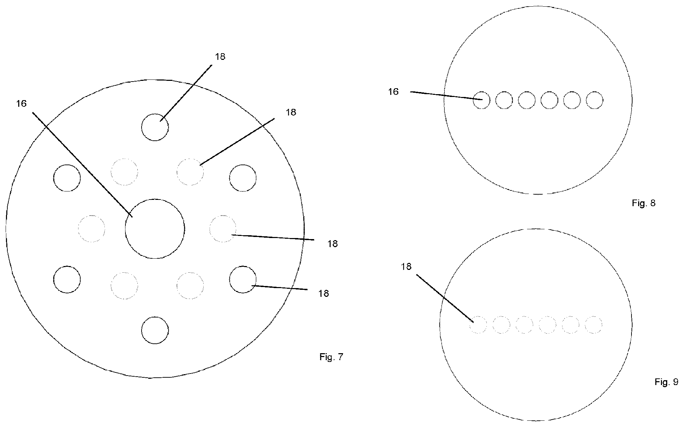

Figures 6 to 9 illustrate alternative arrangements of excitation and detection fibres which could be employed with one or more of the above embodiments of the fetal monitoring device of the invention. Figure 6 illustrates the use of multiple excitation fibres 16 arranged in a circular array surrounding a single centrally positioned detection fibre 18. As detailed above it is preferable that the excitation fibres 16 and the detection fibre 18 are confocal. The positions of the excitation fibres 18 and detection fibre 18 could also be reversed. Figure 7 illustrates a modification to this arrangement in which multiple detection fibres 18 are provided in two concentric circular arrays surrounding a single central excitation fibre 16. As with the arrangement of Figure 6 the positions of the excitation fibre 16 and detection fibres 18 could be reversed. Figures 8 and 9 illustrate configurations in which the excitation fibres 16 and/or the detection fibres 18 are arranged in a linear array.

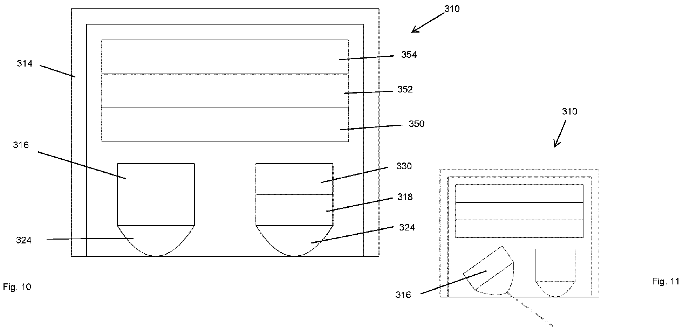

Turning to Figure 10 there is illustrated a fourth embodiment of a fetal monitoring device according to the present invention, generally indicated as 310. In this fourth embodiment like components have been accorded like reference numerals and unless otherwise stated perform a like function. Unlike the previous tethered monitoring devices, the monitoring device 310 is configured to operate untethered or wirelessly, and may therefore be suitably secured to the head of the fetus and left in place for extended periods of time, for example throughout labour, allowing continuous monitoring of fetal wellbeing. The device 310 may therefore comprise means for securing the device 310 to the head of the fetus, for example a suction cup (not shown) or the like circumscribing a lower end of the device 310, vacuum adhesion, passive adhesive, or a combination of one or more of the foregoing. The monitoring device 310 may also comprise means for generating a negative pressure in order to actively adhere the device 310 to the fetal scalp, but is more likely due to space constraints to utilise mechanical generation of a negative pressure by the physical compression of the cup against the scalp during initial application of the device 310. In either case a biocompatible adhesive may additionally or alternatively be employed as hereinbefore described. It will be understood that any other suitable means may be employed to secure the monitoring device 310 at a suitable location relative to the head of the fetus.

The device 310 comprises a hollow housing 314 containing a discrete excitation source 316, for example a laser, which may be covered by a protective and/or focussing lens 324. The excitation source 316 is positioned to emit excitation light from a lower end of the housing 314 onto the head of the fetus. The housing 314 further contains a detection fibre 318 adjacent and preferably confocal with the excitation source 316, and which may also be covered by a protective and/or focussing lens 324. The detection fibre 318 is connected directly to a discrete optical detector 330 such as a charge coupled device (CCD), photodiode or the like and operable to digitally image the reflected light. The monitoring device 310 further comprises electronics 350 necessary to operate the various components of the device 310, a battery 352 to power the device 310, and a radio transmitter 354 to send and/or receive information, most notably to transmit the images captured by the detector 330 to be processed and utilised in providing real time data on the wellbeing of the fetus during labour as hereinbefore described. The device 310 may be modified as shown in Figure 11 such that the excitation source 316 is inclined towards the detection fibre 318 to direct the excitation light towards a target area on the fetal scalp which is directly beneath the end of the detection fibre 318.

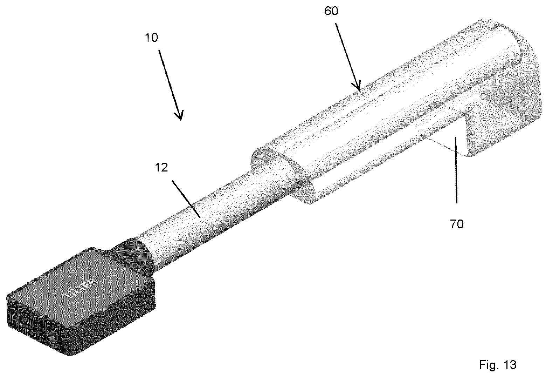

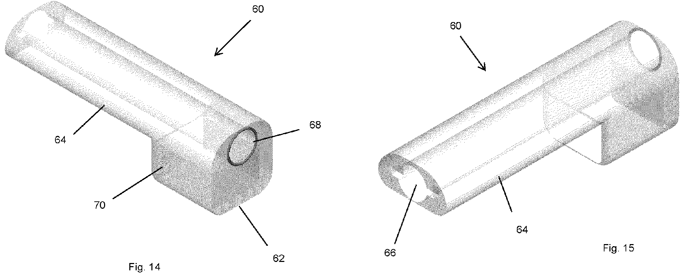

Referring now to Figures 12 to 15 the fetal monitoring device 10 of the invention is illustrated, including an applicator 60 into which the probe 12 is secured, and which is intended to permit intermittent monitoring of fetal wellbeing by allowing the probe 12 to be periodically applied to the head of the fetus by a medical professional during the course of labour. Following each monitoring instance the probe 12 can then be removed. In Figures 12 and 13 the probe 12 is shown in position within the applicator 60 while Figures 14 and 15 show the applicator 60 in isolation. In Figures 12 and 13 an optical filter is connected to the upper end 20 of the probe 12 but it will be understood that this filter is optional and/or the position therefore may be varied.

The applicator 60 comprises a soft or otherwise atraumatic and biocompatible body 62, for example formed from medical grade silicone or the like, having an elongate sleeve 64 defining a lumen 66 for at least partially receiving the probe 12. The lumen 66 terminates at an aperture 68 such that the lower end 22 of the probe 12 is coterminous with the aperture 62. The applicator 60 further defines a chamber or pocket 70 shaped and dimensioned to receive at least a tip of an adult finger such that the applicator 60 may be applied to the finger of the medical professional to allow the probe 12 to be intermittently vaginally introduced and the aperture 68 located against or adjacent the head of the fetus. The probe 12 can then be used as hereinbefore described to scan the vascularised tissue on the head of the fetus in order to monitor various biochemical markers such as lactate levels using Raman spectroscopy.

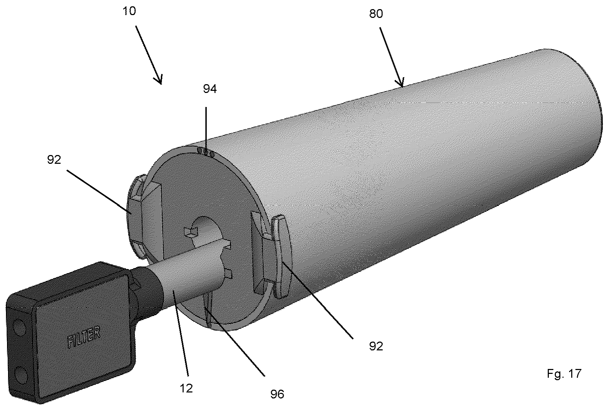

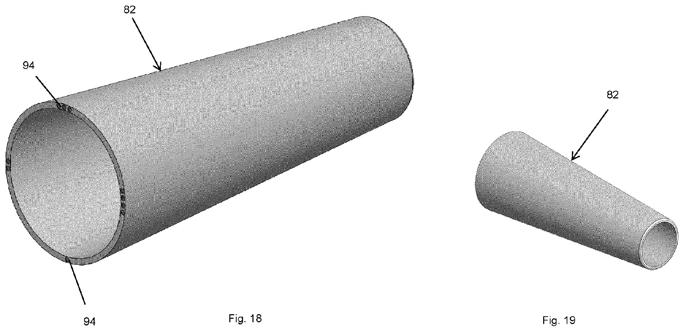

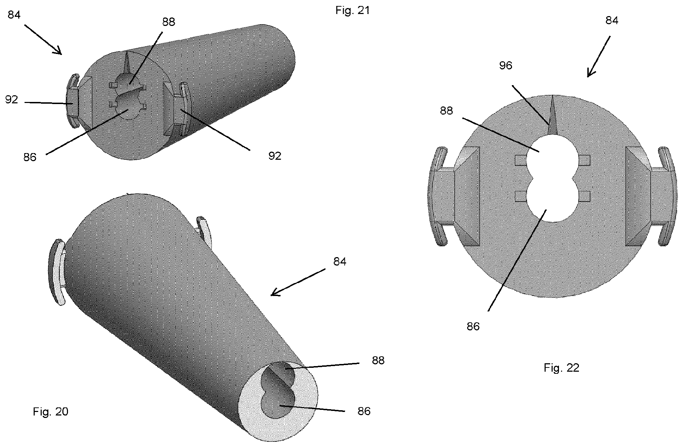

Referring now to Figures 16 to 22 the fetal monitoring device 10 of the invention is illustrated, including an alternative applicator 80 into which the probe 12 is secured, and which is again intended to permit intermittent monitoring of fetal wellbeing by allowing the probe 12 to be periodically applied to the head of the fetus by a medical professional during the course of labour. Following each application the probe 12 is again preferably removed. In Figures 16 and 17 the probe 12 is shown in position within the applicator 80 while Figures 18 to 22 show components of the applicator 80 in isolation. In Figures 16 and 17 an optical filter is connected to the upper end 20 of the probe 12 but it will be understood that this filter is optional and/or the position therefore may be varied.

The applicator 80 comprises an outer sleeve 82 which is conical in shape, and a corresponding conical inner body 84 which is rotatably secured within the outer sleeve 82. The inner body 84 defines at least a first lumen 86 for at least partially receiving the probe 12. A second lumen 88 may be provided offset to the first lumen 86 to allow the probe 12 to be positioned in different orientations. Each of the lumens 86, 88 terminates at an aperture 90 such that the lower end 22 of the probe 12 is coterminous with the aperture 90. The inner body 84 further comprises a pair of lateral tabs 92 which allow the inner body 84 to be gripped and rotated within the outer sleeve 82. In use, by rotating the inner body 84 within the sleeve 82 the focal point of the excitation light can be accurately adjusted as required. The outer sleeve 82 may be provided with a number of positioning indicia 94, for example to indicate ninety degree steps of rotation, with the inner body 84 being provided with a corresponding reference marker 96 to allow the inner body 84 to be accurately rotated into the various relative positions within the outer sleeve 82. With the probe 12 secured in the second lumen 88, which is eccentrically positioned within the outer sleeve 82, rotation of the inner body 84 within the outer sleeve 82 will change the angular position of the lower end 22 relative to the outer sleeve 82. Thus if the outer sleeve 82 is held in a fixed position adjacent the fetal scalp the target area of the scalp onto which excitation light is directed from the lower end 22 can be varied by rotation of the inner body 84. Multiple different readings can therefore be taken, which can mitigate potential issues with using only a single target area which may provide a sub-optimal measurement if for example the target area is contaminated or otherwise obscured. Multiple measurements at different locations on the scalp will avoid any such issues.

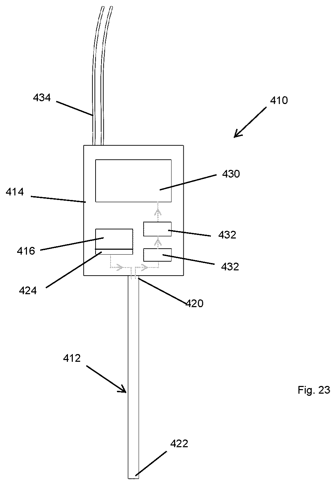

Figure 23 illustrates a fifth embodiment of a fetal monitoring device according to the present invention, generally indicated as 410. In this fifth embodiment like components have been accorded like reference numerals and unless otherwise stated perform a like function. The device 410 comprises a probe 412 extending from a housing 414 which contains a discrete excitation source 416, for example a laser, which may be covered by a protective and/or focussing lens and/or filter 424. The excitation light from the excitation source 416 is directed into the probe 412 which comprises a dual fibre light guide, with one or more excitation fibres (not shown) extending longitudinally from an upper end 420 to a lower end 422 of the probe 412 in order to allow the excitation light to issue from the lower end 422 to be directed, in use, onto the fetal scalp. The probe 412 additionally contains a parallel detection fibre (not shown) again extending longitudinally from the lower end 422 to the upper end 420, to transmit light reflected from the scalp back to the housing 414. The lower end 422 of the probe 412 is preferably covered by a window or lens (not shown).

The monitoring device 410 further comprises a discrete detector 430 which may be in the form of a mini-spectrometer, SiPM, SPAD or CMOS sensor. Upstream of the detector 430 are one or more optical components 432 such as filters which may be long pass or bandpass optical filters. The light reflected from the fetal scalp is therefore passed through the optical components 432 to the spectrometer 430 which is operable to provide local detection and processing of the Raman signals. Electrical connectors 434 provide power and/or data connections to the monitoring device 410, for example allowing the output to be displayed on an external console or the like on which the real time analysis can be displayed. It will however be understood that one or more wireless connections may be provided to the monitoring device 410.

Any of the above described embodiments of the fetal monitoring device of the invention may additionally comprises a temperature sensor (not shown) which may be optically based sensor, a thermistor, or any other suitable component or assembly of components and which may be arranged to be in close or direct contact, in use, with the fetal scalp. The temperature sensor may additionally or alternatively be configured for monitoring heat flow (area temperature measurement). As an alternative to a thermistor the temperature sensor may comprise for example a Mid Infrared (MIR) non-contact sensor to measure temperature. An accurate measurement of temperature for the fetus is an important diagnostic parameter which is not currently measured in real time but when combined with pH and lactate may enhance the real time clinical assessment of fetal well-being. High temperatures (pyrexia) during labour may aggravate an existing hypoxia due to increased tissue oxygen consumption and a shift of the oxygen dissociation curve to the right. Furthermore, the risk of cerebral palsy increases in children born by mothers with clinical signs of chorioamnionitis and fever.

The present invention thus provides a multimodal fetal monitoring device which may be used continuously or intermittently by a clinician to monitor fetal wellbeing during labour. The use of an optical probe and a novel data processing the algorithm the monitoring device can non-invasively extract, in real time, lactate and pH signals from Raman spectra obtained from excitation light reflected off the fetial scalp. The monitoring device and method of the invention therefore allows for measuring of fetal pH and lactate during labour to provide clinicians with a significantly improved, non-invasive means by which to monitor the fetus for issues such as hypoxia.

## Claims

1. A fetal monitoring device comprising an optical emitter operable to emit excitation light at a wavelength of between 500nm-900nm onto vascularised tissue on the head of a fetus; an optical detector operable to detect Raman signals in the reflected light that are generated through Raman scattering of the excitation light; and a processor operable to convert the Raman signal data from the reflected light into correlated real time data regarding one or more biochemical markers to enable an assessment of fetal wellbeing during labour.

2. A fetal monitoring device according to claim 1 in which the optical emitter and/or the optical detector comprise one or more optical fibres.

3. A fetal monitoring device according to claim 2 in which the emitter and/or the detector optical fibres are arranged in an array.
3. A fetal monitoring device according to any preceding claim comprising one or more lenses arranged to focus the excitation light onto a capillary bed of the vascularised tissue.

4. A fetal monitoring device according to any preceding claim comprising one or more optical deflectors arranged to direct the excitation light towards the vascularised tissue.

5. A fetal monitoring device according to any preceding claim comprising an optical shield arranged to isolate the detector from background electromagnetic radiation.

6. A fetal monitoring device according to any preceding claim comprising one or more filters arranged to filter the excitation light and/or the reflected light.

7. A fetal monitoring device according to any preceding claim comprising a camera arranged to image the vascularised tissue on the head of the fetus.

8. A fetal monitoring device according to any preceding claim comprising a housing at least partially containing the optical emitter and optical detector.

9. A fetal monitoring device according to any preceding claim comprising one or more sensors operable to measure one or more of temperature, heat flow, SpO2, PCO2, PO2, and heartrate.

10. A fetal monitoring device according to any preceding claim in which the processor is operable to convert the Raman signal data from the reflected light into measurements of one or more of blood lactate level and blood pH level.

11. A fetal monitoring device according to any preceding claim in which the processor utilises an algorithm to convert the Raman signal data into the correlated real time data regarding the one or more biological markers.

12. A fetal monitoring device according to claim 11 in which the algorithm is arranged to effect background correction of the Raman signals.

13. A fetal monitoring device according to claim 12 in which the background correction is effected using one or more of lock-in detection, time-resolved spectroscopy, multi-wavelength excitation and data processing for lactate quantification.

14. A fetal monitoring device according to any preceding claim in which the optical detector is operable to additionally detect autoflourescence of NAD(P)H in the vascularised tissue; and the processor is operable to convert the autoflourescence data from the reflected light into correlated real time data regarding one or more of the biochemical markers.

15. A method of in-utero monitoring of fetal levels of selected biochemical markers, the method comprising the steps of emitting excitation light at a wavelength of between 500nm-900nm onto vascularised tissue on the head of a fetus; detecting Raman signals that are generated through Raman scattering of the excitation light in light reflected from the vascularised tissue; and converting the Raman signal data from the reflected light into correlated real time data regarding one or more biological markers.

16. A method according to claim 15 comprising the step of converting the Raman signal data from the reflected light into measurements of one or more of blood lactate level, blood pH level, blood oxygen saturation, blood carbon dioxide level, body temperature, hydration and heart rate.

17. A method according to claim 15 or 16 comprising focussing the excitation light onto a capillary bed of the vascularised tissue.

18. A method according to any of claims 15 to 17 comprising emitting the excitation light from a plurality of locations and/or detecting the reflected light from a plurality of locations.

19. A method according to any of claims 15 to 18 comprising passing the excitation light and/or the reflected light through one or more filters.

20. A method according to any of claims 15 to 19 comprising the step of utilising an algorithm to convert the Raman signal data into the correlated real time data regarding the one or more biological markers.

21. A method according to any of claims 15 to 20 comprising the step of effecting background correction of the Raman signals.

22. A method according to claim 21 in which the background correction is effected by using one or more of lock-in detection, time-resolved spectroscopy, multi-wavelength excitation and data processing for lactate quantification.

23. A method according to any of claims 15 to 22 comprising the step of using the optical detector to additionally detect autoflourescence of NAD(P)H in the vascularised tissue; and converting the autoflourescence data from the reflected light into correlated real time data regarding one or more of the biochemical markers.
